# EUROPEAN PATENT SPECIFICATION

(11) **EP 4 066 833 B1**
(45) Date of publication and mention of the grant of the patent: **08.04.2026**
(21) Application number: 20894208.6
(22) Date of filing: 26.11.2020
(51) Int. Cl.: A61K 31/4706, C07D 215/54, A61P 35/00

(54) **SUTETINIB FOR USE FOR THE TREATMENT OF RARE MUTATION-MEDIATED CANCER**
SUTETINIB ZUR BEHANDLUNG VON DURCH SELTENE MUTATION VERMITTELTEM KREBS
SUTETINIB DESTINÉ À ÊTRE UTILISÉ POUR LE TRAITEMENT D'UN CANCER À MÉDIATION PAR MUTATION RARE

(30) Priority: 27.11.2019 CN 201911179729
(43) Date of publication of application: 05.10.2022
(73) Proprietor: Suzhong Pharmaceutical Group Co., Ltd., Taizhou, Jiangsu 225500 (CN); Jiangsu Suzhong Pharmaceutical Research Institute Co., Ltd., Nanjing, Jiangsu 210000 (CN)
(72) Inventor: TANG, Haitao, Nanjing, Jiangsu 210000 (CN); GE, Haitao, Nanjing, Jiangsu 210000 (CN); YI, Nianhong, Nanjing, Jiangsu 210000 (CN); ZHANG, Yuqiang, Nanjing, Jiangsu 210000 (CN); CAO, Sumin, Nanjing, Jiangsu 210000 (CN)
(74) Representative: HGF
(86) International application number: PCT/CN2020/131638
(87) International publication number: WO 2021/104341

(56) References cited:
- WO-A1-2021/104340
- WO-A2-2010/151710
- CN-A- 102 625 797
- CN-A- 104 513 200

## Description

### TECHNICAL FIELD

The present application relates to the field of medical technologies, and more particularly, to an application of substituted butenamide.

### BACKGROUND

Non-small cell lung cancer (NSCLC) is a malignant tumor seriously threatening human health. Although surgery and chemotherapy technologies are continuously improving, patients still have poor prognosis, with a 5-year survival rate less than 20%. At present, molecular targeted therapy with a target spot of epithelium growth factor recptor (EGFR) has become the most important way to treat the NSCLC.

The EGFR is an expression product of a proto-oncogene C-erbB-1, and the gene is located on a No. 7 chromosome and belongs to a transmembrane receptor tyrosine kinase. After binding the EGFR with a ligand thereof, a downstream signal pathway can be activated, and the proliferation, differentiation, angiogenesis and apoptosis inhibition of tumor cells can be regulated, thus controlling a series of tumor biological behaviors.

At present, a clinically used targeted drug against the EGFR is an EGFR tyrosine kinase inhibitor (EGFR-TKI), and the EGFR-TKI blocks an EGFR signal transduction pathway by inhibiting phosphorylation of the EGFR itself, thus inhibiting the proliferation and differentiation of the tumor cells, and realizing targeted therapy.

EGFR mutation may occur in any part of an EGFR sequence. Generally, an EGFR mutant strain is derived from a mutation in a kinase domain (which is namely exons 18 to 24 in the EGFR sequence) or an extracellular domain (which is namely exons 2 to 16 in the EGFR sequence). One or more point mutations in the exon 18 comprise L688P, V689M, P694L/S, N700D, L703V, E709K/Q/A/G/V, I715S, L718P, G719C/A/S/R or S720P/F. Deletions in the exon 19 comprise delG719, delE746_E749, delE746_A750, delE746_A750insRP, delE746_A750insQP, delE746_T751, delE746_T751insA/I/V, delE746_T751insVA, delE746_S752, delE746_S752insA/V/D, delE746_P53insLS, delL747_E749, delL747_A750, delL747_A750insP, delL747_T751, delL747_T751insP/S/Q, delL747_T751insPI, delL747_S752, delL747_S752insQ, delL747_P753, delL747 _P753insS/Q, delL747 _L754insSR, delE749_A750, delE749_A750insRP, delE749_T751, delT751_I759, delT751_I759insS/N or delS752_I759. Duplications in the exon 19 comprise K739_I44dupKIPVAI. Point mutations in the exon 19 comprise L730F, W731Stop, P733L, G735S, V742A, E746V/K, A750P, T7511, S752Y, P753S, A754P or D761Y. In-frame insertions in the exon 20 comprise D761_E762insEAFQ, A767_S768insTLA, V769_D770insY, V769 _D770insCV, V769_D770insASV, D770_N771insD/G, D770_N771insNPG, D770_N771insSVQ, P772_H773insN/V, P772_H773insYNP or V774_C775insHV. Deletions in the exon 20 comprise delM766_A767, delM766_A767insAI, delA767_V769, delD770 or delP772_H773insNP. Duplications in the exon 20 comprise S768_D770dupSVD, A767_V769dupASV or H773dupH. Point mutations in the exon 20 comprise D761N, A763V, V765A/M, S768I, V769L/M, S768I, P772R, N771T, H773R/Y/L, V774M, R776G/H/C, G779S/F, T783A, T784F, L792P, L798H/F, T790M, R803W, K806E or L814P). Point mutations in the exon 21 comprise G810S, N826S, L833V, H835L, L838V, A839T, K846R, T847I, H850N, V851I/A, I853T, L858M/R, A859T, L861Q/R, G863D, A864T, E866K or G873E.

A curative effect of the EGFR-TKI is closely related to an EGFR gene mutation, and the EGFR gene mutation mainly concentrates on the exons 18 to 21, comprising a sensitive mutation and a drug-resistant mutation.

In patients suffering from the lung cancer in China, an EGFR mutation rate accounts for 30% to 50%, wherein mutation rates of the exons 19 and 21 (L858R and L861I) account for about 90% of a total mutation rate, a mutation rate of the exon 18 accounts for about 5% of the total mutation rate, and T790M mutation on the exon 20 accounts for about 5% of the mutation rate. Mutations except for the deletions of the exon 19 and the L858R mutation are called rare mutations, such as L861Q, G719X and S768I.

CN102625797A discloses an effect of (E)-N-(3-cyano-7-ethoxy-4-(3-ethynylphenylamino)quinoline-6-yl)-4-(dimethylamino)butyl-2-enamide on common EGFR mutants such as an EGFR mutant L858R/T790M and an EGFR mutant E746-A750, but does not record an effect on rare mutations such asL861Q, G719X and S768I.

It is clear that a new method for inhibiting cells with the rare EGFR mutations (such as L861Q, G719X and S768I) is needed, and a new therapy for treating cancers related to the mutations will have far-reaching benefits.

### SUMMARY

The invention is defined by the appended claims.

Any references in the description to methods of treatment refer to the compounds, pharmaceutical compositions and medicaments of the present invention for use in a method for treatment of the human (or animal) body by therapy (or for diagnosis).

The present invention aims to provide an application of substituted butenamide, and specifically provides a new application of (E)-N-(3-cyano-7-ethoxy-4-(3-ethynylphenylamino)quinoline-6-yl)-4-(dimethylamino)butyl-2-enamidemaleate and a solvent compound thereof.

The compound (E)-N-(3-cyano-7-ethoxy-4-(3-ethynylphenylamino)quinoline-6-yl)-4-(dimethylamino)butyl-2-enamidemaleate and the solvent compound thereof in the present invention have a good inhibitory activity to cancers mediated by the rare EGFR mutations L861Q, G719X and S768I.

The compound (E)-N-(3-cyano-7-ethoxy-4-(3-ethynylphenylamino)quinoline-6-yl)-4-(dimethylamino)butyl-2-enamide has a structural formula as follows:

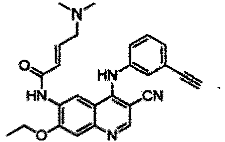

The compound (E)-N-(3-cyano-7-ethoxy-4-(3-ethynylphenylamino)quinoline-6-yl)-4-(dimethylamino)butyl-2-enamidemaleate and the solvent compound thereof in the present invention may inhibit proliferation of cells expressing EGFR mutant strains.

Further, the present invention provides an application of the (E)-N-(3-cyano-7-ethoxy-4-(3-ethynylphenylamino)quinoline-6-yl)-4-(dimethylamino)butyl-2-enamidemaleate and the solvent compound thereof in preparing a drug for treating a rare EGFR mutation-mediated cancer.

The rare EGFR mutation in the present invention is a mutation except for deletions of an exon 19 of an EGFR and an L858R mutation, and comprises any one or a combination of several of EGFR mutants L861Q, G719X and S768I, and the rare EGFR mutation may be a sensitive mutation and a drug-resistant mutation.

The "EGFR mutation-mediated cancer" in the present invention refers to a tumor characterized by an EGFR gene mutation (comprising specific mutations mentioned herein) that changes a biological activity of a nucleic acid molecule or a polypeptide of the EGFR. The EGFR-mediated tumor may appear in any tissue, comprising a brain, blood, a connective tissue, a liver, a mouth, a muscle, a spleen, a stomach, a testis and a trachea. The EGFR-mediated cancer comprises a non-small cell lung cancer (NSCLC), comprises one or more of a squamous cell carcinoma, an adenocarcinoma, a bronchioloalveolar carcinoma (BAC) and a focal invasive BAC, an adenocarcinoma with BAC characteristics and a large cell carcinoma; a neural tumor, such as a glioblastoma; a pancreatic cancer; a head and neck cancer (such as the squamous cell carcinoma); a breast cancer; a colorectal cancer; an epithelioma, comprising the squamous cell carcinoma; an ovarian cancer; a prostate cancer; and the adenocarcinoma; and comprises the EGFR-mediated cancer.

The EGFR-mediated cancer in the present invention is further the non-small cell lung cancer.

The "inhibit proliferation of cells expressing EGFR mutant strains" refers to moderately slowing down, stopping or reversing a growth rate of cells expressing the EGFR in vitro or in vivo. Ideally, when measured by a suitable method for measuring the growth rate of cells (such as a cell growth measurement method described herein), the growth rate is slowed down by at least 10%, 20%, 30%, 50% or even 70%. The EGFR mutant strain may be any EGFR mutant strain described herein. The compound is the maleate, or a hydrate thereof, such as the monohydrate. In some embodiments, the maleate is formed by a conventional method. The maleate is formed by allowing a free alkali form of the compound to react with the following pharmaceutically acceptable acid: a maleic acid Similarly, the compound and the maleate salt thereof or a hemihydrate, a monohydrate, a dihydrate, a trihydrate and a multihydrate thereof are also comprised herein.

When administrated to a patient, the compound (E)-N-(3-cyano-7-ethoxy-4-(3-ethynylphenylamino)quinoline-6-yl)-4-(dimethylamino)butyl-2-enamidemaleate and the solvent compound thereof in the present invention forms a drub composition with a pharmaceutically acceptable adjuvant, and prepared into a suitable drug preparation for administration.

In the drug composition used in the present invention, the pharmaceutically acceptable adjuvant comprises a carrier, an excipient, a binder, a filler, a suspending agent, an aromatic agent, a sweetener, a disintegrant, a dispersant, a surfactant, a lubricant, a colorant, a diluent, a solubilizer, a humectant, a plasticizer, a stabilizer, a penetration enhancer, a wetting agent, a defoamer, an antioxidant, a preservative, or a combination of one or more of them. The drug composition is beneficial for administrating the compound to an organism. When a treatment or use method provided herein is implemented, the therapeutically effective amount of the compound described herein is administered in the form of the drug composition to a mammal suffering from a disease, a symptom or an illness condition to be treated. In some embodiments, the mammal is a human. The therapeutically effective amount may be changed greatly with a severity of disease, age and relative health of individual, an efficacy of used compound and other factors. The compound may be used separately or used in combination with one or more therapeutic agents as a component of a mixture.

The drug preparation in the present invention comprises, but is not limited to an aqueous liquid dispersion, a self-emulsifying dispersion, a solid solution, a liposome dispersion, an aerosol, a solid dosage form, a powder, a quick-release preparation, a controlled-release preparation, a disintegrating (fastmelt) preparation, a tablet, a capsule, a pill, a delayed-release preparation, an extended-release preparation, a pulse-release preparation, a multi-particle preparation, and a mixed quick-release and controlled-release preparation.

An amount of the compound administered to the patient and a dosage regimen of the compound and/or the composition in the present invention for treating the cancer depend on many factors, comprising age, weight, sex and medical condition of individual, a type of disease, a severity of disease, a route and frequency of administration and a specific compound used. Therefore, the dosage regimen may be changed greatly, but can be routinely determined by a standard method. In some embodiments, a daily dosage of about 0.01 mg/kg to 500 mg/kg by weight, preferably a daily dosage of about 0.01 mg/kg to 50 mg/kg by weight, further preferably a daily dosage of about 0.01 mg/kg to 30 mg/kg by weight, and further preferably a daily dosage of about 0.1 mg/kg to 10 mg/kg by weight, and even further preferably a daily dosage of about 0.5 mg/kg to 3 mg/kg by weight are appropriate, and should be available for all use methods disclosed herein. The daily dosage may be administered once to four times per day. In some embodiments, the therapeutically effective amount administrated to the patient is 50 mg to 250 mg, and preferably 100 mg once per day, and is continuously administrated for 28 days.

A suitable administration route for administration to the patient comprises, but is not limited to oral administration, intravenous administration, rectal administration, aerosol administration, parenteral administration, eye administration, lung administration, transmucosal administration, transdermal administration, vaginal administration, ear administration, nasal administration and topical administration. In addition, for example only, the arenteral administration comprises intramuscular injection, subcutaneous injection, intravenous injection and intramedullary injection, and intrathecal injection, direct intraventricular injection, intraperitoneal injection, lymphatic injection and intranasal injection.

The compound (E)-N-(3-cyano-7-ethoxy-4-(3-ethynylphenylamino)quinoline-6-yl)-4-(dimethylamino)butyl-2-enamidemaleate and the solvent compound thereof in the present invention have an outstanding therapeutic effect on the rare EGFR mutation-mediated cancer, and different from a common compound having a therapeutic effect on the rare EGFR mutation-mediated cancer inferior to a therapeutic effect on a non-common non-rare EGFR mutation-mediated cancer, the compound of the present invention has a better therapeutic effect on a non-rare EGRR mutation, such as a T790M or L858R/T790M mutation.

### DETAILED DESCRIPTION

The present invention is further described in combination with the following embodiments, but these embodiments are not intended to limit the scope of the present invention.

(E)-N-(3-cyano-7-ethoxy-4-(3-ethynylphenylamino)quinoline-6-yl)-4-(dimethylamino)butyl-2-enamide involved in an embodiment of the present application was prepared by the method of WO2010151710, and a maleate monohydrate of the (E)-N-(3-cyano-7-ethoxy-4-(3-ethynylphenylamino)quinoline-6-yl)-4-(dimethylamino)butyl-2-enamide was prepared by the method of CN104513200A, which was named a maleate of the (E)-N-(3-cyano-7-ethoxy-4-(3-ethynylphenylamino)quinoline-6-yl)-4-(dimethylamino)butyl-2-enamide.

### Embodiment 1 Measurement of IC50 to protein kinase

A compound measured in the embodiment was (E)-N-(3-cyano-7-ethoxy-4-(3-ethynylphenylamino)quinoline-6-yl)-4-(dimethylamino)butyl-2-enamide.

### 1. Experimental design

IC50 of the compound to the protein kinase was tested, 10 semilog concentrations (1×10⁻⁰⁶M to 3×10⁻¹¹M) were set for a subject, with a single well, and the IC50 was measured.

### 2. Subject

The subject was two tubes of solid, and transferred by ProQinase to -20°C for subsequent use.

Before the test, a stock solution of 1× 10⁻⁰³M subject was prepared with 100% DMSO and further diluted to 1×10⁻⁰⁴M/100% DMSO.

The subject was semi-logarithmically diluted with 100% DMSO into a 96-well plate, and prepared from 1×10⁻⁰⁴M to 3×10⁻⁰⁹M. Before use, the subject was diluted with water at a ratio of 1: 10 to obtain subject samples of 1×10⁻⁰⁵M to 3 ×10⁻¹⁰M, containing 10% DMSO.

5 µl of the subject sample of each concentration was added in the test (see a protein kinase test in Part 3), and a final volume of the test was 50 µl. The subject was measured at the concentration ranging from 1×10⁻⁰⁶M to 3×10⁻¹¹M. A final concentration of DMSO in all reaction wells was 1%.

### 3. Protein kinase test

Activities of 8 protein kinases were measured by a radiometric protein kinase test (³³PanQinase^{®} activity measurement method). All tests were carried out in a Perkin Elmer (Boston, MA, USA) 96-well plate with a reaction volume of 50 µl. Reagents and samples were added by four steps:
10 µl of non-radioactive ATP aqueous solution;
25 µl of buffer/[γ⁻³³P]-ATP mixed solution;
5 µl of subject, containing 10% DMSO; and
10 µl of enzyme/substrate mixture.

The substrate was Poly (Glu, Tyr) 4:1, with a code of SIG_20K5903.

All tests comprised 70 mM HEPES-NaOH at pH 7.5, 3 mM MgCl₂, 3 mM MnC, 3 µM sodium orthovanadate, 1.2 mM DTT, ATP (indefinite, related to apparent ATP-Km of each enzyme, see Table 1), [γ-33P]-ATP (about 8×1,005 cpm per well), protein kinase (indefinite, referring to Table 1), and the substrate (indefinite, referring to Table 1).

Concentrations of enzymes and substrates used in the tests are shown in Table 1:

**Table 1 Concentrations of enzymes and substrates used**

| Serial number | Kinase Name | Concentration of kinase Ng/50 µl | Concentration of kinase nM* | Concentration of APT µM | Substrate Ng/50 µl |
|---|---|---|---|---|---|
| 1 | EGF-R G719C | 20 | 4.5 | 1.0 | 0.125 |
| 2 | EGF-R G719S | 10 | 2.2 | 0.3 | 0.125 |
| 3 | EGF-R L861Q | 25 | 5.6 | 0.3 | 0.125 |
| 4 | EGF-R 790M | 10 | 2.2 | 0.3 | 0.125 |
| 5 | EGF-R T790M/L858R | 10 | 2.2 | 0.3 | 0.125 |
| 6 | EGF-R wt | 5 | 1.1 | 0.3 | 0.125 |
| 7 | ERBB2 | 100 | 21.3 | 1.0 | 0.125 |
| 8 | ERBB4 | 5 | 1.0 | 0.3 | 0.125 |

A reaction system was incubated at 39°C for 60 minutes. The reaction was stopped with 50 µl of 2% (v/v) H₃PO₄, and the plate was washed with 200 µl of 0.9% (w/v) NaCl twice. Enzyme activity-dependent transfer of ³³Pi (calculated by "cpm") was measured by a micro-well plate scintillation counter (Microbeta,Wallac).

All tests were carried out by a BeckmanCoulterBiomek 2000/SL robot.

### 4. Data analysis

For a blank control, three reaction wells were set, without adding the enzyme, and a median of cpm values of the three reaction holes was taken. This value reflected radioactive nonspecific binding in the absence of protein kinase and substrate. In addition, three reaction wells were set, without adding the subject, and a median of cpm values of the three reaction holes was taken, which was a negative control, that was, an enzyme activity without any inhibitor. A difference value between the negative control and the blank control was 100% activity of each enzyme.

As with the negative control from which a blank value was subtracted, the blank value should be subtracted from the values of the corresponding 10 subjects. A residual enzyme activity of each well was calculated according to the following formula: residual enzyme activity (%) = 100×[ (cpm value of the subject - blank control)/(negative control - blank control)].

10 concentrations were set for each subject to act on each enzyme, so that the data analysis was based on these 10 residual enzyme activity values. S-curve fitting was carried out to calculate the IC50 according to a fixed maximum value of 100% and a fixed minimum value of 0% by using Prism5.04 (Graphpad, San Diego, California, USA www.graphpad.com).

### 5. Results

IC50 values of the tested compound acting on eight kinases are listed in Table 2.

**Table 2 IC50 values of the tested compound acting on eight kinases**

| Serial number | Kinase | IC50 (M) |
|---|---|---|
| 1 | EGF-R G719C | 1.40E-09 |
| 2 | EGF-R G719S | 1.50E-09 |
| 3 | EGF-R L861Q | 1.90E-09 |
| 4 | EGF-R 790M | 1.20E-07 |
| 5 | EGF-R T790M/L858R | 8.80E-08 |
| 6 | EGF-R wt | 1.30E-09 |
| 7 | ERBB2 | 2.70E-08 |
| 8 | ERBB4 | 1.00E-08 |

The above data show that an inhibitory effect of the tested compound on rare mutations is better than that of the tested compound on common non-rare mutations.

### Embodiment 2 Exploratory clinical research test for treatment of locally advanced or metastatic non-small cell lung cancer

### I. Selection standards of subjects

### 1. Selection standards:

(1) The patients are 18 years old to 75 years old (including 18 years old and 75 years old), regardless of sex.
(2) The patients suffer from locally advanced or metastatic NSCLC, which is confirmed by histopathology and/or cytology.
(3) The EGFR of the patients has one or more of L861Q, G719X and S768I mutations, but does not have a T790M mutation, a deletion mutation of an exon 19, an insertion mutation of an exon 20 and an L858R mutation.
(4) The patients have an ECOG physical strength score of 0, 1 or 2.
(5) The patients have an expected survival time greater than three months.
(6) The patients have at least one evaluable tumor focus according to the response evaluation criteria in solid tumor (RECIST) 1.1.
(7) The patients have sufficient blood system function, liver function, kidney function and coagulation function:
   absolute count of neutrophils ≥ 1.5×109/L, count of platelets ≥ 90×109AL, hemoglobin ≥ 90 g/L;
   total bilirubin ≤ 1.5×upper limit of normal value (ULN), alanine aminotransferase (ALT) ≤ 2.5×ULN, and aspartate transaminase (AST) ≤ 2.5×ULN (for patients suffering from liver metastasis, total bilirubin ≤ 3.0×ULN, ALT ≤ 5.0×ULN, AST ≤ 5.0× ULN);
   creatinine ≤ 1.0×ULN, or creatinine clearance ≥ 60 mL/min (by a Cockcroft-Gault method); and
   international normalized ratio (INR) ≤ 1.5;
(8) The qualified fertile patients (male and female) must agree to use a reliable contraceptive method (a hormone or barrier method, or abstinence) during the test period and at least 90 days after the last administration; the female patients of childbearing age must be negative in a pregnancy test of human chorionic gonadotropin (HCG) within seven days before being selected; and the male patients cannot donate sperm from the first administration to 90 days after the last administration.
(9) All patients must know about this research before starting any examination specified in this test, and voluntarily sign a written informed consent form (ICF) approved by the ethics committee.

### 2. Exclusion standards:

(1) The patients received any anti-tumor treatment with an epidermal growth factor receptor tyrosine kinase inhibitor (EGFR-TKI) before joining the group.
(2) The patients who received chemotherapy, anti-angiogenesis and other systemic anti-tumor treatments in the past need an elution period of four weeks and more; the patients who received anti-PD-1/PD-L1 and other immunotherapies in the past without suffering from immune pneumonia need an elution period of four weeks and more; the patients who received radiotherapy on non-target focuses for the purpose of relieving symptoms and anti-tumor Chinese patent medicine treatment need an elution period of two weeks and more.
(3) The patients received a major organ surgery (excluding puncture biopsy) or had a significant trauma within four weeks before joining the group.
(4) At the time of screening, an adverse reaction (ADR) of any previous treatment in the past has not recovered to grade evaluation less than or equal to a grade 1 in the common terminology criteria for adverse events (CTCAE) 5.0 (except alopecia).
(5) The patients are unable to take a drug orally, suffer from a serious (the grade evaluation in the CTCAE 5.0 is greater than or equal to a grade 3) chronic gastrointestinal dysfunction, and have a malabsorption syndrome or any other conditions affecting gastrointestinal absorption (such as peptic ulcer, intestinal obstruction, an irritable bowel syndrome, a Crohn's disease and a gastroesophageal reflux disease).
(6) The patients have an immunodeficiency, comprising, but being not limited to a positive result in a human immunodeficiency virus (HIV) antibody detection (enzyme-linked immunosorbent assay or Western dot assay), and an active rheumatic immune disease.
(7) The patients suffer from unstable central nervous system metastasis or meningeal metastasis with clinical symptoms, or there are other evidences that a focus of the central nervous system metastasis or the meningeal metastasis of the patients has not been controlled, and it is judged by researchers that the patients are not suitable for joining the group; and the patients with clinically suspected brain or pia mater lesions should be excluded by a computed tomography/magnetic resonance imaging (CT/MRI) detection.
(8) The patients have a history of severe (the grade evaluation in the CTCAE 5.0 is greater than or equal to the grade 3) bullous or exfoliative skin disease.
(9) At the time of screening, the patients have an uncontrolled active infection [such as a syphilis, HIV, hepatitis B virus (being positive in HBsAg, HBV-DNA> 1000 cps/ml and AST/ALT > 2.0xULN) or hepatitis C virus (HCV) infection].
(10) The patients have a history of severe [heart function grading of New York Heart Association (NYHA) is a grade III or a grade IV] cardiovascular disease, comprising, but being not limited to ventricular arrhythmia requiring clinical intervention; the patients had an acute coronary syndrome, a congestive heart failure, cerebral apoplexy or other cardiovascular events of grade III or above within six months before joining the group; and at the time screening, the heart function grading of NYHA is greater than or equal to a grade II or a left ventricular ejection fraction (LVEF) is less than 50%.
(11) The patients have histories of other severe (the grade evaluation in the CTCAE 5.0 is greater than or equal to the grade 3) systemic diseases, and it is judged by researchers that the patients are not suitable for participating in the clinical test.
(12) The patients participated in other clinical tests within four weeks before joining the group.
(13) It is known that the patients have alcohol or drug dependence.
(14) The patients suffer from a mental disorder, or researchers think that the patients with poor compliance are not suitable for participating in the research.
(15) The patients are pregnant or lactating women.
(16) It is known that the patients are allergic to active ingredients or excipients of the test drug.
(17) The patients need long-term treatment with adrenal steroid hormone (equivalent to daily dosage of prednisolone greater than or equal to 20 mg, see Appendix IV for a dosage conversion table).
(18) The patients suffered from other malignant tumors in the past five years or at the same time (except the patients whose skin basal cell carcinoma and carcinoma in situ of cervix were cured; and the patients whose breast cancer did not recur within more than three years after radical mastectomy).

### II. Administration method and dosage

A maleate monohydrate capsule of the (E)-N-(3-cyano-7-ethoxy-4-(3-ethynylphenylamino)quinoline-6-yl)-4-(dimethylamino)butyl-2-enamide (referring to Chinese patent application 201911180660.1) is taken orally at least one hour before or two hours after a meal, the dosage is 100 mg once a day, and the treatment lasts for 28 days. The administration starts every four weeks as a cycle.

### III. Therapeutic effects

**Table 3 Sum of longest diameters of target focuses at the ends of treatment cycles**

| Serial number of subject | Sex | Type of gene mutation | Sum of longest diameters of all target focuses | | | Rate of change |
|---|---|---|---|---|---|---|
| | | | Screening period | The end of second cycle | The end of fourth cycle | |
| 001 | Female | L861Q | 65.8 mm | 35 mm | 30.1 mm | -54% |
| 002 | Female | L861Q | 58.6 mm | 40.4 mm | 36.7 mm | -37% |
| 003 | Female | G719X | 20.8 mm | 16 mm | 13.6 mm | -35% |
| 004 | Female | G719X and S768I | 105.0 mm | 48.0 mm | 43.0 mm | -59% |
| 005 | Male | S768I | 62.8 mm | 38.7 mm | 39.6 mm | -37% |
| 006 | Male | G719X | 81.0 mm | 31.0 mm | 34.8 mm | -57% |
| 007 | Male | S768I | 89.1 mm | 54.8 mm | 57.3 mm | -38% |
| 008 | Female | G719X | 23.9 mm | 14.7 mm | 18.2 mm | -38% |
| 009 | Female | L861Q and G719X | 88.4 mm | 51.6 mm | 47.9 mm | -46% |
| 010 | Female | L861Q | 18.0 mm | 8.9 mm | | -51% |

**Table 4 Sum of shortest diameters of target focuses during treatment cycles**

| Serial number of subject | Sex | Type of gene mutation | Sum of long diameters in screening period | Sum of shortest long diameters | Therapeutic effect evaluation | PFS (month) |
|---|---|---|---|---|---|---|
| 001 | Female | L861Q | 65.8 mm | 29.9 mm | PR | 8 |
| 002 | Female | L861Q | 58.6 mm | 36.7 mm | PR | 14 |
| 003 | Female | G719X | 20.8 mm | 13.6 mm | PR | 14 |
| 004 | Female | G719X and S768I | 105.0 mm | 41.0 mm | PR | 13 |
| 005 | Male | S768I | 62.8 mm | 38.7 mm | PR | 10 |
| 006 | Male | G719X | 81.0 mm | 27.0 mm | PR | 12 |
| 007 | Male | S768I | 89.1 mm | 54.8 mm | PR | 12 |
| 008 | Female | G719X | 23.9 mm | 13.5 mm | PR | 11 |
| 009 | Female | L861Q and G719X | 88.4 mm | 47.3 mm | PR | 9 |

| | | | | | | |
|---|---|---|---|---|---|---|
| PFS: progression free survival | | | | | | |

The above treatment results show that patients suffering from cancers mediated by rare EGFR mutations all show good therapeutic effects after treatment.

## Claims

1. (E)-N-(3-cyano-7-ethoxy-4-(3-ethynylphenylamino)quinoline-6-yl)-4-(dimethylamino)butyl-2-enamide maleate, or a solvent compound thereof for use in treatment of a rare epithelium growth factor receptor (EGFR) mutation-mediated cancer; wherein the rare EGFR mutation comprises any one or a combination of several of EGFR mutants L861 Q, G719X and S768I.

2. E)-N-(3-cyano-7-ethoxy-4-(3-ethynylphenylamino)quinoline-6-yl)-4-(dimethylamino)butyl-2-enamide maleate or a solvent compound thereof for use according to claim 1, wherein the solvent compound is a hydrate.

3. E)-N-(3-cyano-7-ethoxy-4-(3-ethynylphenylamino)quinoline-6-yl)-4-(dimethylamino)butyl-2-enamide maleate or a solvent compound thereof for use according to claim 1, wherein the solvent compound is a hemihydrate and a monohydrate.

4. E)-N-(3-cyano-7-ethoxy-4-(3-ethynylphenylamino)quinoline-6-yl)-4-(dimethylamino)butyl-2-enamide maleate or a solvent compound thereof for use according to claim 1, wherein the cancer is a non-small cell lung cancer.

5. (E)-N-(3-cyano-7-ethoxy-4-(3-ethynylphenylamino)quinoline-6-yl)-4-(dimethylamino)butyl-2-enamide maleate or a solvent compound thereof for use according to claim 1 in the amount of 50 mg to 250 mg per day, and preferably 100 mg once per day.

6. (E)-N-(3-cyano-7-ethoxy-4-(3-ethynylphenylamino)quinoline-6-yl)-4-(dimethylamino)butyl-2-enamide maleate or a solvent compound thereof for use according to claim 1 in the amount of 0.01 mg/kg to 500 mg/kg, preferably 0.01 mg/kg to 50 mg/kg, further 0.01 mg/kg to 30 mg/kg, and further 0.1 mg/kg to 10 mg/kg or 0.5 mg/kg to 3 mg/kg by weight.

## Patentansprüche

1. (E)-N-(3-Cyano-7-ethoxy-4-(3-ethynylphenylamino)chinolin-6-yl)-4-(dimethylamino)butyl-2-enamidmaleat oder Lösungsmittelverbindung davon zur Verwendung bei Behandlung eines durch seltene Epithelwachstumsfaktorrezeptor(EGFR-)Mutation vermittelten Krebses; wobei die seltene EGFR-Mutation eine beliebige oder eine Kombination aus mehreren von EGFR-Mutanten L861 Q, G719X und S768I umfasst.

2. (E)-N-(3-Cyano-7-ethoxy-4-(3-ethynylphenylamino)chinolin-6-yl)-4-(dimethylamino)butyl-2-enamidmaleat oder Lösungsmittelverbindung davon zur Verwendung nach Anspruch 1, wobei die Lösungsmittelverbindung ein Hydrat ist.

3. (E)-N-(3-Cyano-7-ethoxy-4-(3-ethynylphenylamino)chinolin-6-yl)-4-(dimethylamino)butyl-2-enamidmaleat oder Lösungsmittelverbindung davon zur Verwendung nach Anspruch 1, wobei die Lösungsmittelverbindung ein Hemihydrat und ein Monohydrat ist.

4. (E)-N-(3-Cyano-7-ethoxy-4-(3-ethynylphenylamino)chinolin-6-yl)-4-(dimethylamino)butyl-2-enamidmaleat oder Lösungsmittelverbindung davon zur Verwendung nach Anspruch 1, wobei der Krebs ein nichtkleinzelliger Lungenkrebs ist.

5. (E)-N-(3-Cyano-7-ethoxy-4-(3-ethynylphenylamino)chinolin-6-yl)-4-(dimethylamino)butyl-2-enamidmaleat oder Lösungsmittelverbindung davon zur Verwendung nach Anspruch 1 in der Menge von 50 mg bis 250 mg pro Tag und bevorzugt 100 mg einmal pro Tag.

6. (E)-N-(3-Cyano-7-ethoxy-4-(3-ethynylphenylamino)chinolin-6-yl)-4-(dimethylamino)butyl-2-enamidmaleat oder Lösungsmittelverbindung davon zur Verwendung nach Anspruch 1 in der Menge von 0,01 mg/kg bis 500 mg/kg, bevorzugt 0,01 mg/kg bis 50 mg/kg, ferner 0,01 mg/kg bis 30 mg/kg und ferner 0,1 mg/kg bis 10 mg/kg oder 0,5 mg/kg bis 3 mg/kg nach Gewicht.

## Revendications

1. Maléate de (E)-N-(3-cyano-7-éthoxy-4-(3-éthynylphénylamino)quinoléine-6-yl)-4-(diméthylamino)butyl-2-énamide, ou composé solvant de celui-ci destiné à être utilisé dans le traitement d'un cancer médié par une mutation rare du récepteur du facteur de croissance épidermique (EGFR) ; ladite mutation rare de l'EGFR comprenant l'un quelconque ou une combinaison de plusieurs mutants EGFR L861Q, G719X et S768I.

2. Maléate de (E)-N-(3-cyano-7-éthoxy-4-(3-éthynylphénylamino)quinoléine-6-yl)-4-(diméthylamino)butyl-2-énamide ou composé solvant de celui-ci destiné à être utilisé selon la revendication 1, ledit composé solvant étant un hydrate.

3. Maléate de (E)-N-(3-cyano-7-éthoxy-4-(3-éthynylphénylamino)quinoléine-6-yl)-4-(diméthylamino)butyl-2-énamide ou composé solvant de celui-ci destiné à être utilisé selon la revendication 1, ledit composé solvant étant un hémihydrate et un monohydrate.

4. Maléate de (E)-N-(3-cyano-7-éthoxy-4-(3-éthynylphénylamino)quinoléine-6-yl)-4-(diméthylamino)butyl-2-énamide ou composé solvant de celui-ci destiné à être utilisé selon la revendication 1, ledit cancer étant un cancer du poumon non à petites cellules.

5. Maléate de (E)-N-(3-cyano-7-éthoxy-4-(3-éthynylphénylamino)quinoléine-6-yl)-4-(diméthylamino)butyl-2-énamide ou composé solvant de celui-ci destiné à être utilisé selon la revendication 1 en une quantité de 50 mg à 250 mg par jour, et de préférence 100 mg une fois par jour.

6. Maléate de (E)-N-(3-cyano-7-éthoxy-4-(3-éthynylphénylamino)quinoléine-6-yl)-4-(diméthylamino)butyl-2-énamide ou composé solvant de celui-ci destiné à être utilisé selon la revendication 1 en une quantité de 0,01 mg/kg à 500 mg/kg, de préférence 0,01 mg/kg à 50 mg/kg, en outre 0,01 mg/kg à 30 mg/kg, et en outre 0,1 mg/kg à 10 mg/kg ou 0,5 mg/kg à 3 mg/kg en poids.
